# EUROPEAN PATENT APPLICATION

(11) **EP 3 643 724 A1**
(43) Date of publication of application: **29.04.2020**
(21) Application number: 19209309.4
(22) Date of filing: 09.07.2014
(51) Int. Cl.: C07K 16/06, C07K 16/08, C07K 16/10, C07K 16/12, A61K 39/00

(54) **CANINE HEALTH PRODUCT CONTAINING ANTIBODIES AGAINST CANINE PARVOVIRUS TYPE 2**

(30) Priority: 09.07.2013 EP 13305976; 13.03.2014 GB 201404503
(62) Divisional of application: 14736847.6
(71) Applicant: Mars, Incorporated, McLean, VA 22101 (US)
(72) Inventor: FEUGIER, Alexandre, 30470 AIMARGUES (FR); CHASTANT, Sylvie, 30470 AIMARGUES (FR); MILA, Hanna, 30470 AIMARGUES (FR); GRELLET, Aurélien, 30470 AIMARGUES (FR)
(74) Representative: Nony

(57) **Abstract**

The present invention relates to composition containing antibodies from hyperimmunized egg powder against Canine parvovirus type 2 in combination with one of the following antibodies from a hyperimmunized egg powder against;
Canine coronavirus,
*E.coli,*
Canine Herpesvirus,
optionally also in combination with an antibody from a hyperimmunized egg powder against
*Bordetella bronchiseptica* or
*Giardial,*
or comprising antibodies from a hyperimmunized egg powder against one or more of the following;
Canine parainfluenza virus,
Canine distemper virus,
Adenovirus type 1,
*Streptococci,*
*Staphylococci or*
*Isospora,*
for use orally in a treatment to improve dog health, administered within the first 24 hours of age.

## Description

### Field of the Invention

The present invention relates to design and manufacture of integrated circuits and more particularly to electrical modelling of integrated circuits combining high voltage power devices with low voltage control logic blocks, and even more particularly, the modelling of substrate coupling effects in these circuits.

### Background

Automotive applications requires more and more integration of high voltage (HV) power devices together with low voltage (LV) control logic blocks. This integration on a single substrate lead to the development of a new category of integrated circuit (IC), often called Smart Power ICs.

In a typical Smart Power IC, the power switch, the control logic and sensor circuit operate at different voltage levels and different temperatures. In such devices, induced substrate coupling noise becomes a critical issue due to switching of the power stage. During switching, substrate parasitic currents consisting of electrons and holes lead to a local shift of the substrate potential that can reach hundreds of millivolts. This is due to the presence of inductive loads which can cause forward/reverse biasing of diode junctions inside substrate. As a result, current paths driven by propagation of electrons and holes are collected by nearby sensitive analog and digital devices, even at long distance.

Minority carriers are injected into the substrate during switching of high voltage power stages, and propagated inside substrate. Collected current may cause malfunction of sensitive nearby low voltage devices and sometimes may be destructive due to the presence of triggered latch up. This is the major cause of failure and cost circuit redesign, since simulations cannot predict them.

For instance, the parasitic lateral NPN between a 50V NDMos and a low voltage NMOS transistor is difficult to characterize, because its electrical characteristics are dependent on the distance between the two devices which is layout dependent. This distance corresponds to the substrate base of the parasitic BJT.

Due to substrate noise coupling, the number of failures is now increasing. Industry reports that for 10 to 15 years lifetime product chips, these have globally more than 10 versions of the chips during development and fabrication.

Nowadays, standard parasitic extraction tools consider only the parasitic effect within device.

However, the commutation between devices (e.g. parasitic current injected from high voltage transistor and collected at low voltage transistor) due to minority carriers propagation is often neglected and hard to model since parasitic lateral bipolar between devices cannot be extracted from industrial tools. Numerous attempt to evaluate the substrate coupling noise from high-voltage devices was done by numerical solutions relying on semiconductor physicals behaviours. However, experimental extraction methodology based on predicting a simple equivalent circuit from circuit layout is not convenient for a complex layout design and lack of accuracy.

Technology Computer-Aided-Design (TCAD) software are today the only tools available to evaluate the impact of minority carriers propagation. However, TCAD simulations can only be applied to a few discrete devices, since it applies finite-element method for numerically solving physical equations in 2D and 3D structures. This is usually time-consuming, in range of hours, depending on the complexity of layout structure. Thus, there is still no suitable solution to model the substrate coupling noise for automotive application.

In 2010, a novel model methodology has been proposed in order to take into account the minority carriers propagtion within circuit simulators. It consists in the representation of the substrate with an equivalent parasitic network of diodes and resistances. This allow to track substrate coupling noise for a general circuit layout.

However, there is still the problem of generating the equivalent network which models accurately the substrate behavior, the network being as simple as possible in order to reduce the computing load of the electrical simulation.

### Summary

Therefore, the aim of the proposed method is to obtain automatically an electrical network accurately modelling the parasitic effects in the substrate of the studied integrated circuit.

In a first embodiment, a method for generating an electrical circuit comprising at least a network of diodes, resistors and/or homojunctions, said electrical circuit modellising the parasitic effects in a substrate of an integrated circuit comprising electronic devices, said integrated circuit being defined by a set of mask layer layouts and a technology rules file, comprises:
- generating a simplified 3D layout of said integrated circuit from the set of mask layer layouts and the technology rules file by using only mask layers associated to technology layers involved in the parasitic effects;
- defining in the simplified layout a plurality of internal regions, each internal region corresponding to one electronic device of the integrated circuit and one external region corresponding to the part of the simplified 3D layout not included in any internal region;
- computing in parallel and independently:
   - for each internal region, a 3D matrix of adjacent rectangular cuboids, such that there is a limit between at least two adjacent rectangular cuboids where there is a change of doping type or where there is a change of doping concentration in the simplified layout;
   - for the external region, a mesh of adjacent rectangular cuboids with no overlap and no gap;
- extracting a parasitic component with two terminals between each pair of adjacent cuboids, each terminal being positioned at the center of one of the two adjacent cuboids, such as:
   - if the two adjacent cuboids have two different doping types, defining the parasitic component as a diode; or
   - if the two adjacent cuboids have the same doping type with the same doping concentration, defining the parasitic component as a resistor; or
   - if the two adjacent cuboids have the same doping type and different concentrations, defining the parasitic component as a homojunction; and
   - defining the electrical caracteristics of each parasitic component based on the geometry of the adjacent cuboids and the technological parameters; and
- connecting all extracted parasitic components into an electrical circuit by considering each rectangular cuboid center as a node of the parasitic component network.

Advantageously, the method allows to automatically obtain an electronic circuit modelling the parasitic effects in the substrate of the studied integrated circuit. Furthermore, using two different partition technics for internal regions and external region allows to minimize the number of parasitic components in the final electronic circuit. Consequently, the following simulations are much more efficient.

This embodiment may comprises other features, alone or in combination, such as:
- the mask layer layouts used to generate the simplified 3D layout comprise at least the doped substrate layers and the doped contact layers;
- a set of xyz coordinates is applied onto the simplified 3D layout, where x and y coordinates define horizontal planes and z coordinate define the integrated circuit depth;
- the 3D matrix of internal region is computed by:
   - scanning the mask layers for finding and collecting corner points of change in the doping type or the doping concentration;
   - flattening corner points to xy coordinates so that an horizontal rectangular tessellation is built in which each corner point is a corner of at least one rectangle;
   - constructing layers of rectangular cuboids by projecting the rectangular tessellation on the z axis, each layer depth corresponding to the depth of at least one corner point;
- the internal regions are defined by:
   - scanning and detecting the outermost area of each internal regions;
   - collecting the lower left and upper right corner points of each internal regions;
   - defining rectangular cuboid with said lower left and upper right corner points as internal region;
- for the external region, the mesh of rectangular cuboids is computed by:
   - flattening the lower left and upper right corner points of each internal region onto a xy plane;
   - building a rectangular tessellation in which each corner point is a corner of at least one rectangular cell;
   - if a rectangular cell is contained inside any one of the internal region area, flag it as "in region" and do nothing;
   - if not, verify if said rectangular cell can be merged with an adjacent rectangular cell for forming a merged rectangular cell.

In a second embodiment, a digital data storage medium is encoding a machine-executable program of instructions to perform the method disclosed here above.

In a third embodiment, a system for generating an electrical circuit comprising at least a network of diodes, resistors and/or homojunctions, said electrical circuit modellising the parasitic effects in a substrate of an integrated circuit comprising electronic devices, said integrated circuit being defined by a set of mask layer layouts and a technology rules file, comprises:
- a generator of a simplified 3D layout of said integrated circuit from the set of mask layer layouts and the technology rules file by using only mask layers associated to technology layers involved in the parasitic effects;
- means for defining in the simplified 3D layout a plurality of internal regions, each internal region corresponding to one electronic device of the integrated circuit and one external region corresponding to the part of the simplified 3D layout not included in any internal region;
- a computer to compute in parallel and independently:
   - for each internal region, a 3D matrix of adjacent rectangular cuboids, such that there is a limit between at least two adjacent rectangular cuboids where there is a change of doping type or where there is a change of doping concentration in the simplified layout;
   - for the external region, a mesh of adjacent rectangular cuboids with no overlap and no gap;
- an extractor of a parasitic component with two terminals between each pair of adjacent cuboids, each terminal being positioned at the center of one of the two adjacent cuboids, such as:
   - if the two adjacent cuboids have two different doping types, defining the parasitic component as a diode; or
   - if the two adjacent cuboids have the same doping type with the same doping concentration, defining the parasitic component as a resistor; or
   - if the two adjacent cuboids have the same doping type and different concentrations, defining the parasitic component as a homojunction; and
   - defining the electrical caracteristics of each parasitic component based on the geometry of the adjacent cuboids and the technological parameters; and
- a network generator of all extracted parasitic components into an electrical circuit by considering each rectangular cuboid center as a node of the parasitic component network.

### Brief Description of the Figures

Some embodiments of apparatus and/or methods in accordance with embodiments of the present invention are now described, by way of example only, and with reference to the accompagnying drawings, in which :
- The Figure 1 schematically illustrates mask layer layouts of a simple diode;
- The Figure 2 illustrates the same diode in a 3D view;
- The Figure 3 is a flowchart of an embodiment of the method for generating an electrical circuit;
- The Figures 4A to 4F illustrates different steps of a detailed embodiment for creating a mesh of rectangular cuboids of the diode of Figures 1 and 2;
- The Figure 5 is a flowchart of an embodiment of the method for generating a 3D matrix of an internal region;
- The Figure 6A to 6G illustrates different steps of a detailed embodiment for creating a mesh of an external region;
- The Figure 7 is a flowchart of an embodiment of the method for generating a mesh of an external region;
- The Figures 8A and 8B illustrates the component extraction mechanism on the 3D matrix built up at Figures 4A to 4D; and
- The Figure 9 schematically illustrates the caracteristics of adjacent cuboids for defining the electrical parameter of the associated parasitic component.

### Description of Embodiments

During design phase, an integrated circuit is defined by a set of mask layer layouts and a technology rules file. Each mask layer layout is a 3D drawing defining the geometry of a lithography mask of a defined microelectronic manufacturing process. For instance, there is a mask for a polysilicon layer, another mask for a contact layer, etc. During drawing of these mask layers, specific geometric rules associated to a given technology are enforced in order to obtain a coherent stack of layers.

The technology rules file contains the geometric rules, process rules such as the thickness of a given layer and also electrical rules such as the resistivity by square of a given layer.

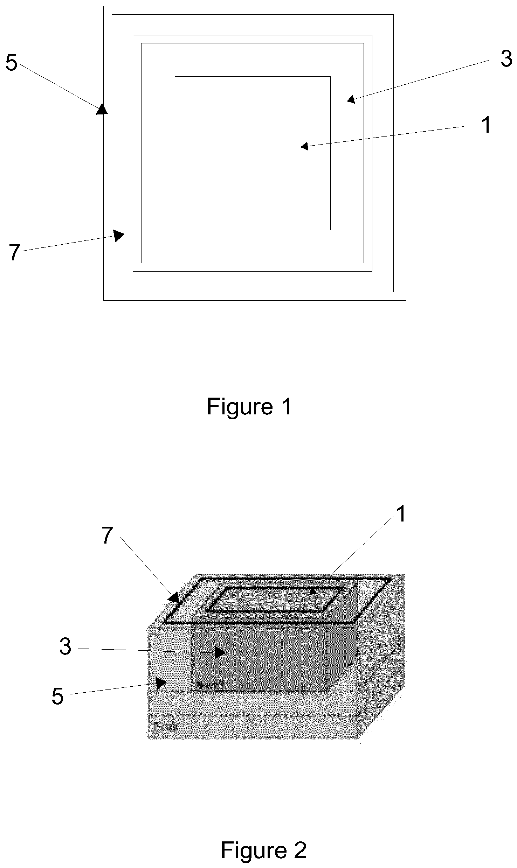

Figure 1 represents an extract of the set of mask layer layouts representing a simple diode. Area 1 is the contact area of a N well 3 which is enclosed by a P substrate 5 having a contact area 7.

Figure 2 represents the same diode in a pseudo 3D view to show the relative thickness of each layer.

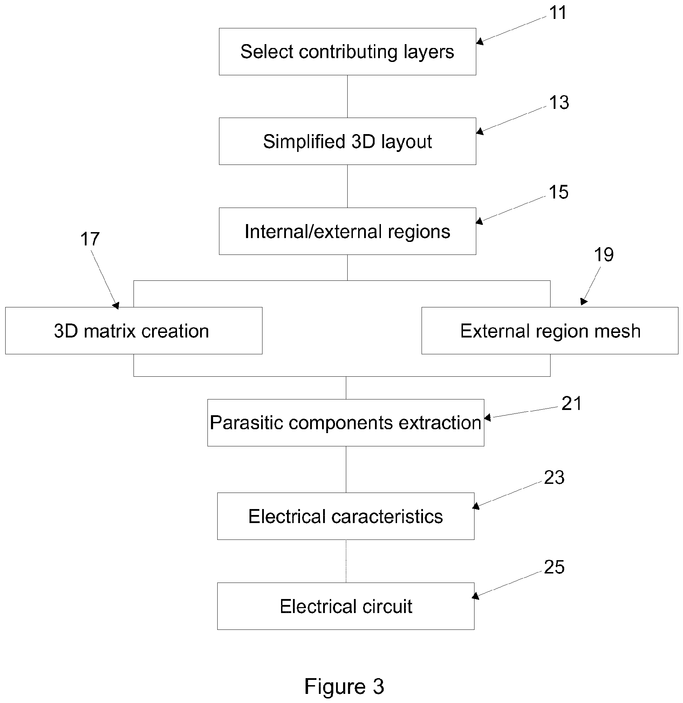

As the aim of the disclosed embodiment is to modelize the parasitic effects in a substrate of an integrated circuit, Figure 3, only mask layers associated to technology layers involved in the parasitic effects are considered, step 11. For instance, contact layers and doping layers are considered but not the metal layers used to connect the different electronic components. In the example of the diode, the considered layers are the N well layer and the P+ and N+ layers of the contact area. These layers are called contributing layers.

From the considered set of mask layer layouts and the technology rules file, a simplified 3D layout similar to Figure 2 is generated, step 13.

At step 15, in the 3D simplified layout, a plurality of internal regions is defined. Each internal region corresponds to one electronic device of the integrated circuit. And one external region corresponding to the part of the simplified 3D layout not included in any internal region is also defined. The external region is thus the complementary region of all internal regions in the 3D layout.

Then, in parallel and independently,
- for each internal region, a 3D matrix of adjacent rectangular cuboids is computed, step 17, such that there is a limit between at least two adjacent rectangular cuboids where there is a change of doping type or where there is a change of doping concentration in the simplified layout;
- for the external region, a mesh of adjacent rectangular cuboids with no overlap and no gap is computed, step 19.

For both types of region, there are some common rules for building the 3D matrix or the mesh:
- The basic building block is a rectangle cuboid, i.e. a cuboid having 6 faces and all angles are right angles and opposite faces of a cuboid are equal ;
- A rectangle cuboid contains only one type of layer, i.e. same doping type and same doping concentration ;
- A consequence of the previous rule is that the interface between two doping types, for instance, correspond to faces of cuboids.
- The cuboids define a partition of each region in the mathematical sense of a partition of a set : every element of a region is in exactly one of these cuboids ; and
- Two cuboids are said adjacent when they have a face in common.

Detailed embodiments of steps 17 and 19 are disclosed after the general description of the present embodiment.

At step 21, a parasitic component with two terminals is extracted for each pair of adjacent cuboids, each terminal being positioned at the center of one of the two adjacent cuboids, such as:
- if the two adjacent cuboids have two different doping types, the parasitic component is a diode; or
- if the two adjacent cuboids have the same doping type with the same doping concentration, the parasitic component is a resistor; or
- if the two adjacent cuboids have the same doping type and different concentrations, the parasitic component is a homojunction.

When the parasitic component is a diode, the orientation of the diode is defined by the doping type of each cuboids.

A homojunction is defined here only as a semiconductor interface that occurs between layers of similar semiconductor material having the same doping type, either N type or P type, but with different doping level.

At step 23, the electrical caracteristics of each parasitic component is computed based on the geometry of the adjacent cuboids and the technological parameters.

For instance, the value of a resistance will be the product of the distance between the centers of the two cuboids by the resistivity of the considered layer.

At step 25, all extracted parasitic components are connected into an electrical circuit by considering each rectangular cuboid center as a node of the parasitic component network.

The electrical circuit is then ready to be inputted into an electrical simulator such as SPICE for analyzing its electrical behavior and particularly the impact of parasitic effects of the substrate.

In the following embodiments, a xyz coordinate system is defined where xy define a plane parallel to the surface of the integrated circuit and z defines its depth.

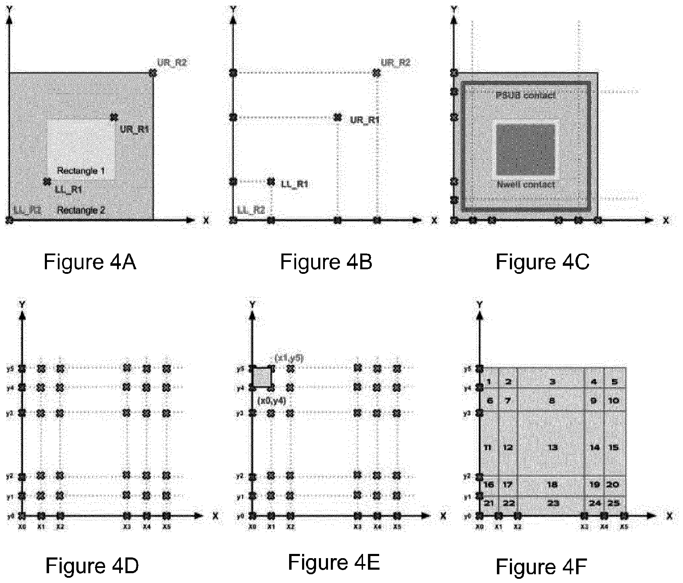

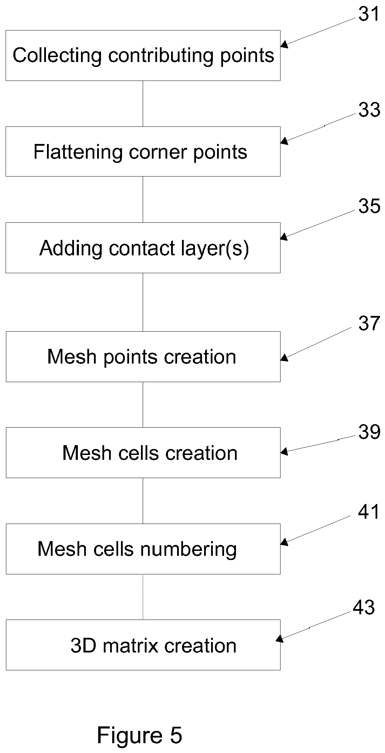

A detailed internal meshing strategy corresponding to step 17 is now disclosed, Figures 4A to 4F and Figure 5.

The internal meshing mechanism is based on collecting contributing corner points. The main steps are:
- Colllection of contributing points: by scanning the contributing layers, the extraction engine finds and collects, step 31, automatically the corner points of geometrical shapes as represented in Figure 4A. For example, the upper right point UR_R1 and lower left point LL_R1 of rectangle 1.
- Flattening corner points to xy mesh coordinate array: Figures 4B shows that these coordinates are projected, step 33, onto a 2D xy plane, i.e. the z coordinate is set temporarely to 0, corresponding to the top surface. Then, Figure 4C the contact layer is added, step 35. The mesh coordinates of the contact layer are computed so that the contact line is on a median of the associated rectangle.
- Each extracted x coordinate combines, step 37, with every extracted y coordinate to represent one mesh point. In the exemple, it means the generation of 36 mesh points, Figure 4D.
- Each 4 adjacent mesh points constructs, step 39, a rectangle mesh cell and two diagonal of them represent the geometrical size of the mesh cell. For instance, Figure 4E, the upper left rectangle is represented by coordinates (x0, y4) and (x1, y5).
- Then each rectangle is numbered consecutively, step 41, from the top left rectangle to the bottom right rectangle, row by row, Figure 4F.
- Then the z coordinates are taken into account to generate a 3D matrix, step 43. The number of cuboid layers and the thickness of each cuboid layer is defined by the different z coordinates of the corner points.

In pseudo code, the meshing algorithm for internal region can be written as:

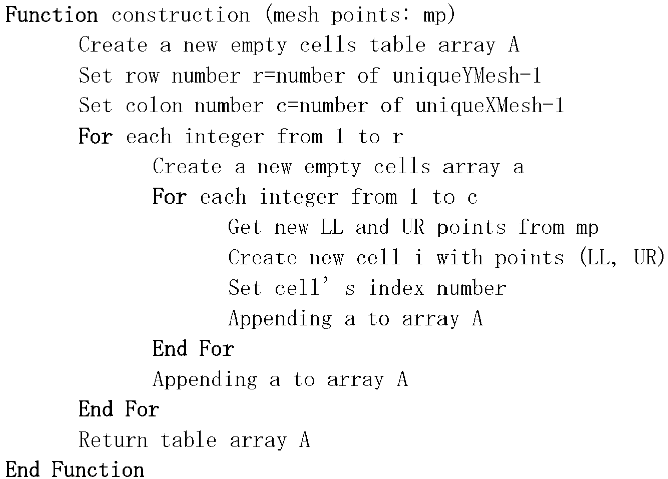

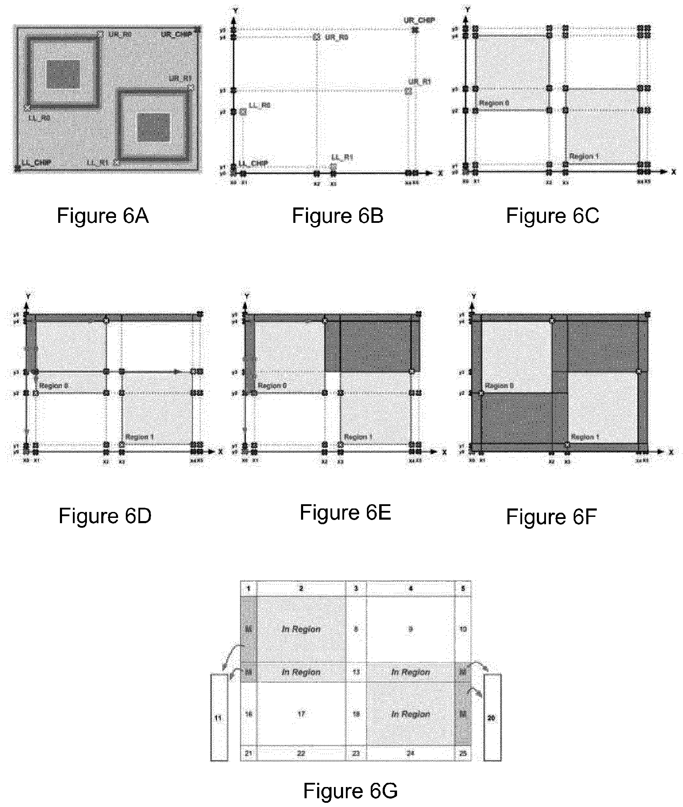

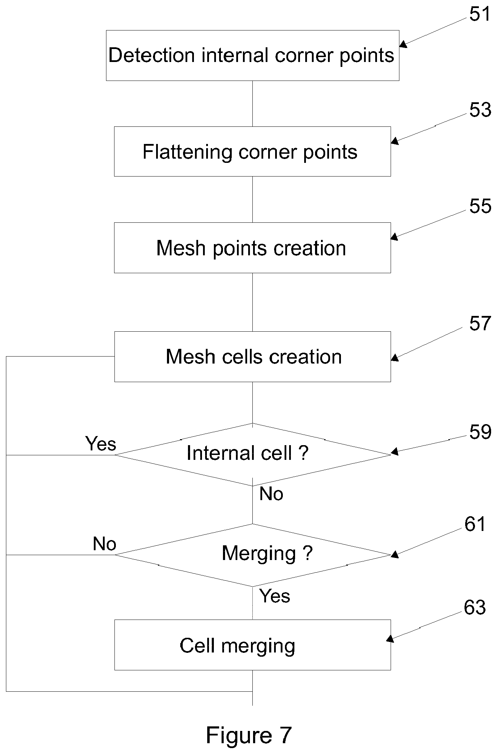

A detailed external meshing strategy corresponding to step 19 is now disclosed, Figures 6A to 6G and Figure 7.

The lower left LL and upper right UR corner points of each internal regions have been previously detected as shown Figure 4A, step 51.

Similarly with the internal meshing strategy disclosed here above, the LL and UR corner points of internal regions are projected onto an xy mesh coordinate plane, constituting a xy mesh coordinate array, step 53.

From this array, and in a process similar to the process generating Figures 4D and 4E, all mesh points are generated, step 55, and mesh cells, step 57.

Then, all mesh cells are scanned for verifying if the mesh cell is contained inside any one of the internal region area, step 59. If it is so, the mesh cell is flagged as « in region » and will not be constructed. Otherwise, each mesh cell is analyzed to determine if it can be merged with adjacent cells,step 61.

If it is possible to merge two adjacent cells, a new cell is created, step 63 by merging the two adjacent cells and the new cell is flagged as « is merged » and contains the synthetized information of both cells.

Figures 6D to 6F illustrate the merging mechanism and Figure 6G illustrates the numbering and flagging techniques.

In pseudo code, the meshing algorithm of external region can be written as :

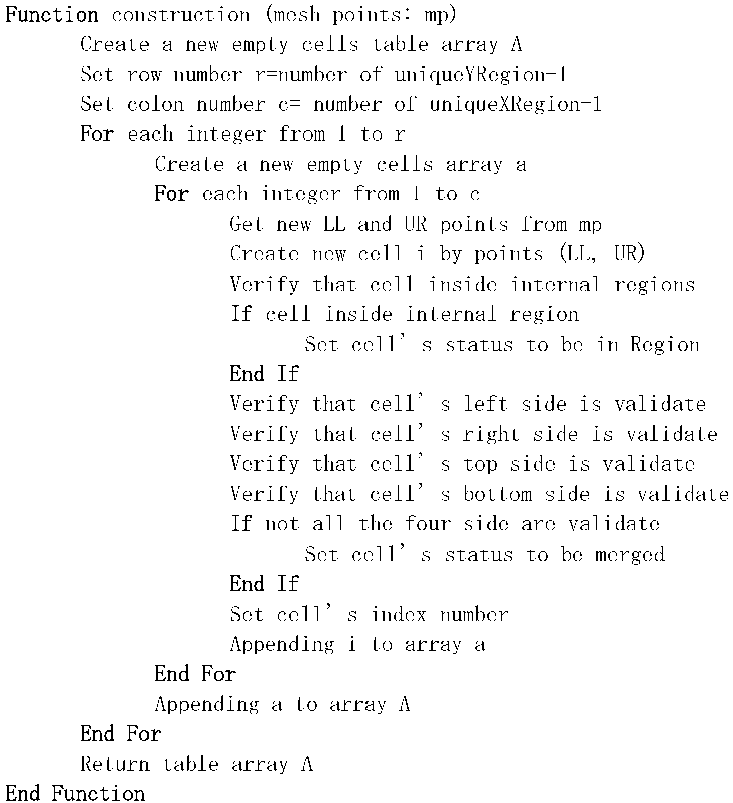

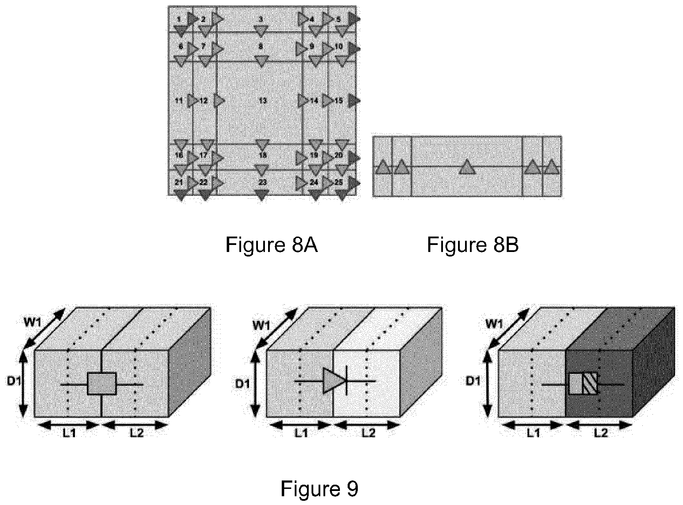

Figures 8A and 8B illustrates the component extraction mechanism on the 3D matrix built up at Figures 4A to 4D.

In the 3D matrix, for each cuboid, lateral adjacent cuboids and vertical adjacent cuboids are considered to define the horizontal and the vertical contributions of the cuboid, the cuboid being a node of the built electrical network.

For instance, on Figure 8A, the cuboid 1 has two adjacent cuboids numbered 2 (at right) and 6 (at bottom).

On vertical contribution, by construction of the 3D matrix, each cuboid has only one adjacent cuboid as shown on Figure 8B.

In a 3D view, Figure 9, a cuboid has length, width and depth. Therefore, each cuboid represents a specific space of the substrate and contains three types of information : technology (doping type), geometry (width, length an depth) and algoritmic (position inside the 3D mesh network).

The following lines illustrate the substrate component extraction algorithm of internal and external regions.

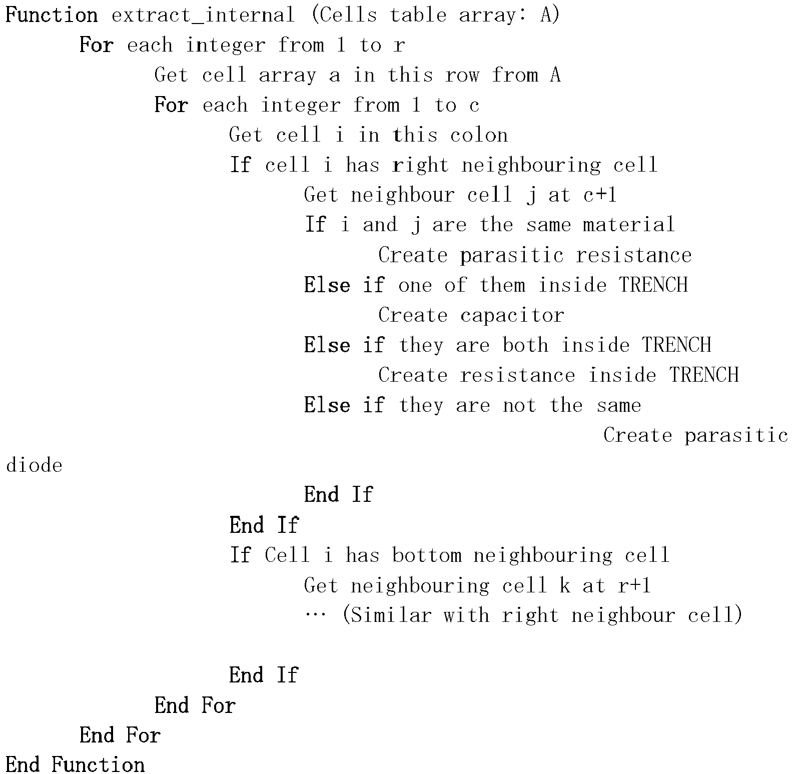

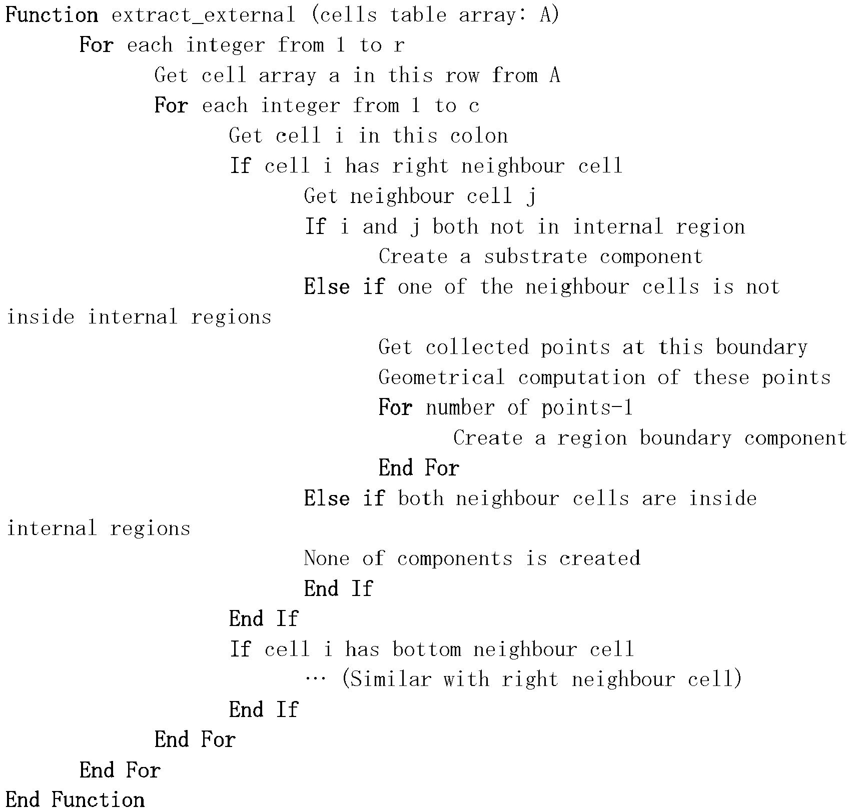

A person of skill in the art would readily recognize that steps of various above-described methods can be performed by programmed computers. Herein, some embodiments are also intended to cover program storage devices, e.g., digital data storage media, which are machine or computer readable and encode machine- executable or computer-executable programs of instructions, wherein said instructions perform some or all of the steps of said above-described methods. The program storage devices may be, e.g., digital memories, magnetic storage media such as a magnetic disks and magnetic tapes, hard drives, or optically readable digital data storage media. The embodiments are also intended to cover computers programmed to perform said steps of the above-described methods.

The functions of the various elements shown in the figures., including any functional blocks labeled as "processors", may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" or "controller" should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), and non volatile storage. Other hardware, conventional and/or custom, may also be included. Similarly, any routers shown in the figures are conceptual only. Their function may be carried out through the operation of program logic, through dedicated logic, through the interaction of program control and dedicated logic, or even manually, the particular technique being selectable by the implementer as more specifically understood from the context.

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

The description and drawings merely illustrate the principles of the invention. It will thus be appreciated that those skilled in the art will be able to devise various arrangements that, although not explicitly described or shown herein, embody the principles of the invention and are included within its spirit and scope. Furthermore, all examples recited herein are principally intended expressly to be only for pedagogical purposes to aid the reader in understanding the principles of the invention and the concepts contributed by the inventor(s) to furthering the art, and are to be construed as being without limitation to such specifically recited examples and conditions. Moreover, all statements herein reciting principles, aspects, and embodiments of the invention, as well as specific examples thereof, are intended to encompass equivalents thereof.

## Claims

1. A composition containing antibodies from hyperimmunized egg powder against Canine parvovirus type 2 in combination with one of the following antibodies from a hyperimmunized egg powder against;
Canine coronavirus,
*E.coli,*
Canine Herpesvirus,
optionally also in combination with an antibody from a hyperimmunized egg powder against
*Bordetella bronchiseptica* or
*Giardial,*
or comprising antibodies from a hyperimmunized egg powder against one or more of the following;
Canine parainfluenza virus,
Canine distemper virus,
Adenovirus type 1,
*Streptococci,*
*Staphylococci or*
*Isospora,*
for use orally in a treatment to improve dog health, administered within the first 24 hours of age.

2. A composition for use as claimed in claim 1, also comprising a hydrolysed carbohydrate selected among maltose, dextrose, maltodextrin or fructose.

3. A composition for use as claimed in claim 1 or claim 2, in the form of a spray, liquid, mousse or gel.

4. A composition for use as claimed in any one of claims 1 to 3, wherein the composition includes antibodies from a hyperimmunized chicken egg against one or more of the virus and against *E. coli.*

5. A composition for use as claimed in any one of claims 1 to 4, wherein the composition comprises antibodies from a hyperimmunized chicken egg against canine coronavirus and/or adenovirus type 1.

6. A composition for use as claimed in any one of claims 1 to 5, wherein the composition is administered twice, at least four hours apart, within the first 24 hours of age.

7. A composition for use as claimed in any of claims 1 to 6, wherein the treatment to improve dog health is a treatment of digestive immunity.

8. A composition for use as claimed in any of claims 1 to 6, wherein the treatment to improve dog health is growth.

9. A composition for use as claimed in any of claims 1 to 6, wherein the treatment to improve dog health is a treatment of stress.

10. A composition for oral use for improving dog health containing antibodies from a hyperimmunized egg powder against Canine parvovirus type 2 in combination with one of the following antibodies from a hyperimmunized egg powder against;
Canine coronavirus,
*E.coli,*
Canine Herpesvirus,
optionally also in combination with an antibody from a hyperimmunized chicken egg against
*Bordetella bronchiseptica or*
*Giardial*
or comprising antibodies from a hyperimmunized chicken egg against one or
more of the following
Canine parainfluenza virus,
Canine distemper virus,
Adenovirus type 1,
*Streptococci,*
*Staphylococci or*
*Isospora,*

11. A composition as claimed in claim 10, also comprising a hydrolysed carbohydrate also comprising a hydrolysed carbohydrate selected among maltose, dextrose, maltodextrin or fructose.
